# EUROPÄISCHE PATENTANMELDUNG

(11) **EP 1 025 821 A1**
(43) Veröffentlichungstag der Anmeldung: **09.08.2000**
(21) Anmeldenummer: 99810089.5
(22) Anmeldetag: 04.02.1999
(51) Int. Cl.: A61F 13/00, D05C 17/00

(54) **Medizinalprodukt mit textilem Bestandteil**

(71) Anmelder: Flawa Schweizer Verbandstoff- und Wattefabriken AG, CH-9230 Flawil (CH); Bischoff Textil AG, 9001 St. Gallen (CH)
(72) Erfinder: Wintermantel, Erich, Prof. Dr., 5442 Fislisbach (CH); Mayer, Jörg, Dr., 5702 Niederlenz (CH); Karamuk, Erdal, 8125 Zollikerberg (CH); Seidl, Roland, Dr., 9630 Wattwil (CH); Wagner, Bärbel, 9008 St. Gallen (CH); Bischoff, Bernhard, 9200 Gossau (CH); Billia, Mario, Dr., 9542 Münchwilen (CH)
(74) Vertreter: Liebetanz, Michael, Dipl.-Phys.

(57) **Zusammenfassung**

Ein Medizinalprodukt mit einem textilen Bestandteil, wie beispielsweise eine Wundkompresse, weist eine Oberfläche (12) auf. Die Oberfläche (12) verfügt eine Vielzahl von Öffnungen (14, 24, 34), die in mindestens zwei Lochmustern angeordnet sind. Der Durchmesser einer Öffnung (14; 24) von einem Lochmuster zu dem Durchmesser einer Öffnung (24; 14 bzw. 34) eines anderen Lochmusters weicht jeweils um ungefähr mindestens einen Faktor 5 voneinander ab. Durch die Anpassung der strukturellen und mechanischen Eigenschaften des Medizinalproduktes an die Eigenschaften des Zielgewebes wird eine bessere Wundversorgung erreicht.

## Beschreibung

Die Erfindung betrifft ein Medizinalprodukt mit einem textilen Bestandteil, wie beispielsweise eine Wundkompresse, mit den Merkmalen des Oberbegriffs des Anspruchs 1.

Im medizinischen Bereich ist eine Reihe von textilen Produkten bekannt, die beispielsweise extern zur Unterstützung der Wundheilung vorgesehen sind. Bekannte Medizinalprodukte wie Wundkompressen bestehen beispielsweise aus Gewebe, welches den Nachteil hat, dass es eine harte und sich der Wunde schlecht anpassende Oberfläche aufweist. Daher sind viele Wundkompressen aus Gestricken aufgebaut, die an sich weich sind. Ferner weisen sie teilweise einen Feuchtigkeitsspeicher auf. Die Weichheit von Gestricken entsteht durch Verschieben der Fäden innerhalb der Bindung. Diese oben genannten Wundprodukte weisen den Nachteil auf, dass sie durch von der Wunde ausgehende Exsudate verhärten und damit ihre Funktionsfähigkeit verlieren.

Bekannte Kompressen weisen semipermeable Membranen aus Polyurethan auf, die den Durchtritt und Austausch von Gasen und Flüssigkeiten gestatten und haben insbesondere nicht haftende Materialien auf der wundseitigen Oberfläche, um das Festkleben an der Wunde zu verhindern. Gerade diese Art von Kompressen sind nicht in der Lage, die Angiogenese gezielt zu fördern, die mit der erfindungsgemässen Oberflächenstruktur gewonnen wird. Die US 5,465,735 beschreibt eine solche vielschichtige Wundabdeckung mit einem dichten Faservlies, welches insbesondere eine geringere Verklebung mit der Wunde ermöglichen soll.

Weitere Einsatzmöglichkeiten von Medizinalprodukten mit einem textilen Bestandteil sind beispielsweise die Behandlung von Bauchwanddefekten im Leistenbereich oder zur Verstärkung von Weichgewebe an anderen Stellen. Eine entsprechende Technik ist in der US 5,569,273 beschrieben, welche eine hexagonale Netzstruktur aus Polypropylen-Monofilament-Garnen beschreibt. Durch die Konstruktion werden grosse Öffnungen zwischen benachbarten vertikalen Maschenreihen erzeugt, in welche Körpergewebe aufgrund der Porenstruktur in das Implantat einwachsen kann. Eine die Angiogenese fördernde Wirkung hat dieses Produkt allerdings nicht.

Aus der EP 870 820 ist ein nicht haftender Wundverband bekannt, der über seinem wirksamen Bereich Vertiefungen besitzt, die eine pharmazeutische Trägersubstanz enthalten. Die Vertiefungen sind lediglich zur Aufnahme und Abgabe eines Wirkstoffes vorgesehen. Das Nichthaften des Wundverbandes wird herausgestellt.

Schliesslich beschreibt die EP 931 012 eine Kompresse zur Wundbehandlung im feuchten Milieu, die ebenfalls durch entsprechende Wahl des Abdeckmaterials nicht an der Wunde anhaften soll.

Ausgehend von diesem Stand der Technik liegt der Erfindung die Aufgabe zugrunde, ein Medizinalprodukt der eingangs genannten Art anzugeben, welches die Angiogenese respektive den damit verbundenen Heilungsvorgang gezielt beeinflusst und fördert.

Ein Ziel der Erfindung liegt auch darin, dass solch ein Medizinalprodukt in seinem textilen Bestandteil auch nach längerem Wundkontakt weiterhin weich bleibt.

Ein weiteres Ziel der Erfindung liegt darin, bei einem solchen Medizinalprodukt die Steifigkeit individuell bei der Herstellung vorgeben zu können.

Schliesslich liegt eine weitere Aufgabe der Erfindung auch darin, die Angiogenese und damit die Geweberegeneration bei Vorliegen beispielsweise eines Ulcus cruris zu verbessern.

Diese Aufgabe wird erfindungsgemäss dadurch gelöst, dass die Oberfläche eine Vielzahl von Öffnungen aufweist, wobei mindestens zwei Lochmuster mit Gruppen von Öffnungen bestehen, wobei der Durchmesser einer Öffnung von einem Lochmuster zu dem Durchmesser einer Öffnung eines anderen Lochmusters jeweils um ungefähr mindestens einen Faktor 5 voneinander abweicht.

Der Vorteil des Einsatzes von Medizinalprodukten gemäss der Erfindung liegt für den Patienten in der rascheren Heilung, in der Reduktion der mit der Wundversorgung verbundenen Schmerzen, in der geringeren Aufenthaltszeit in einer stationären Abteilung und in der für die Volkswirtschaft wesentlichen Kostensenkung für die Behandlung solcher Wunden. Diese Vorteile werden durch die Anpassung der strukturellen und mechanischen Eigenschaften des Medizinalproduktes an die Eigenschaften des Zielgewebes erreicht.

Beim Ulcus cruris liegt der zentrale Punkt in der Wundheilung bei der Regeneration eines physiologisch funktionellen vasculären Systems. Die Wundheilung ist im Zusammenhang mit der Narbengewebsbildung zu sehen. Eine intensive Narbengewebsbildung beeinträchtigt den Patienten aufgrund der schlechten kosmetischen Darstellung und insbesondere aufgrund der beschränkten Mobilität. Beides führt zum persönlichen Unwohlsein und in vielen Fällen zur Invalidität. Wenn die Wunde geheilt ist, besteht ungünstigenfalls ein verbindendes Narbengewebe, bei dem die Kollagenmatrix in dichten parallelen Bündeln rekonstruiert ist, wohingegen das Maschenwerk in unverletzter Haut mechanisch bessere Eigenschaften aufweist.

Die schnelle Vascularisierung kann zu einer unkontrollierten Bildung des Hautkapillarsystems führen. Die Kapillaren selbst beeinflussen die Orientation der Kollagenfibern.

Mechanische Signale in der Gestalt der Ausübung eines kontrollierten Zuges auf die Zellen im Wundgrund können einen wichtigen Aktivator der Wundantwort bilden. Mechanische Einflüsse an der Wunde spielen auch eine Rolle in der Kollagengenese, weil veränderte Spannungen während der Wundschliessung die Vernarbung beeinflussen. Es wird angenommen, dass zur Ausbildung einer normalen Kollagenarchitektur eine definierte physiologische mechanische Stimulation in bezug auf Belastung und Orientation erforderlich ist. Beim Narbengewebe hingegen ist die Anisotropie des Kollagennetzwerkes sowie die Dimensionen der Kollagenfasern erhöht.

Im Gegensatz zu dem beim Stand der Technik herausgestellten Merkmal des Nichthaftens am Wundgrund ist hier das Einsprossen von Gewebe in den Medizinalartikel erwünscht und vorteilhaft.

Die Erfindung schafft ein auf Textilbasis entwickeltes Wundbehandlungssystem, welches die Gewebsbildung steuert und die Angiogenese positiv beeinflusst, indem es als Gerüst wirkt. Dabei ist der Träger der erfindungsgemässen Schicht von der Anwendung abhängig. Die Anwendung der erfindungsgemässen medizinalen Produkte ist in vielen Bereichen möglich. Ein Anwendungsbereich betrifft die Behandlung von grossen Wunden, von Verbrennungen oder in chirurgischen Anwendungen wie beispielsweise für Herniennetze. Diese Verfahren benötigen Behandlungssysteme, die eine Minimierung der Narbenbildung gestatten. Gleichzeitig können in den Medizinalprodukten Mannose-6-Phospat oder andere kollagenregulierende oder geweberegenerationsfördernde Faktoren wie beispielsweise Wachstumsfaktoren der TGF-b-Familie eingebunden werden.

Das Medizinalprodukt kann bei vielen Anwendungen eingesetzt werden, wo die gestickspezifischen Eigenschaften wie die kontrollierten mechanischen Eigenschaften eines Gesticks, die lokale Variation im mechanischen Design sowie die gezielte Porosität von grossem Vorteil sein können. Dies sind neben den erwähnten Kompressen und Herniennetzen der Bauchdeckenersatz, künstliche Blutgefässe und künstliche Bänder. Bei letzteren kann durch die Sticktechnik von einer ersten gezielten Struktur, wo das Band anwachsen soll und wo Lastübertragung stattfindet, zu einer zweiten unterschiedlichen Struktur im Bandbereich übergegangen werden. Eine weitere Anwendung ist die Gestaltung von Augmentationsgesticken zur Rekonstruktion des Kieferknochens im Dentalbereich.

Je nach den gewünschten mechanischen und strukturellen Eigenschaften des Textils werden verschiedene Garntypen verwendet. Bei diesen kann es sich um fasrige, Multifil- oder Monofil-Garne handeln, die zudem unbehandelt, antimikrobisch vorbehandelt, gelbeschichtet und in unterschiedlichen Titern vorliegen können.

Durch den Einsatz der Sticktechnologie sind die Knotengrösse und die Art der Verknüpfung ebenfalls entsprechend voreinstellbar.

Nachfolgend wird die Erfindung anhand von Ausführungsbeispielen näher beschrieben. Es zeigen:
- Fig. 1: eine schematische Querschnittsansicht eines Medizinalartikels gemäss einem Ausführungsbeispiel der Erfindung,
- Fig. 2: eine schematische Draufsicht auf einen Ausschnitt der gestickten Oberfläche eines Medizinalartikels gemäss der Erfindung, und
- Fig. 3: eine vergrösserte Ausschnittsansicht des Bereichs einer Pore nach Fig. 2.

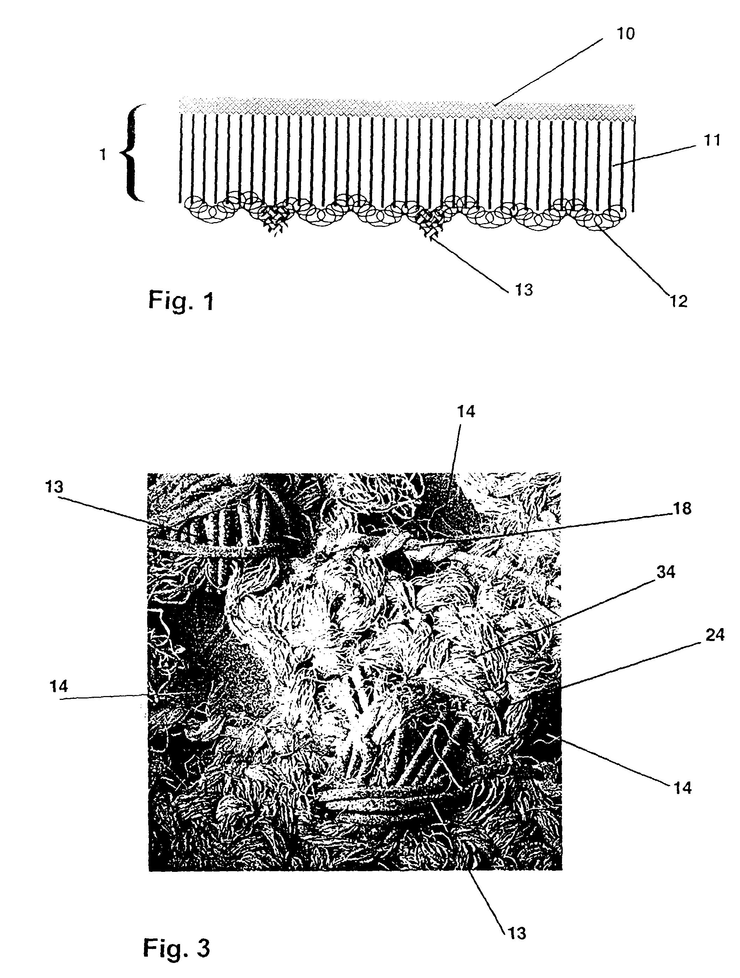

Die Figur 1 zeigt in schematischer Schnittansicht einen Medizinalartikel 1 gemäss der Erfindung. Dieser Medizinalartikel ist beispielsweise aus drei Schichten aufgebaut. Eine wundferne Basisschicht besteht aus dichtem Gewebe 10, welches antibakteriell wirkt. Gleichzeitig geschieht hier die Sauerstoff- und Wassergehaltssteuerung. Eine Abstandschicht 11 mit hoher Scherkraftaufnahme aus gelmodifizierten Garnen gestattet die Absorption und Desorption sowie die mechanische Verbindung des Kompressenmaterials. Die Scherkraftaufnahme gestattet die Verteilung von lokalen Druckbelastungen, das heisst, sie beinhaltet eine Polsterfunktion und gestattet eine Spannungsverteilung, welche zur gleichmässigen Belastung der Wundfläche führt und damit negative lokale Beanspruchungen vermeidet. Schliesslich verfügt die Kompresse 1 über eine gestickte Auflage 12. Das Gewebe 11 ist gegenüber der antibakteriellen Schicht 10 als Distanzhalter und als ein Exsudat aufnehmendes Material ausgestaltet. Auf diesem vorteilhafterweise als Gewirk ausgestaltetem Gewebe 11 liegt die getrennt hergestellte gestickte Auflage 12, die vorzugsweise an ihren seitlichen Rändern mit der Kompresse 1 beispielsweise durch Schallschweissung verbunden ist. Mit dem Bezugszeichen 13 sind Reizpunkte bezeichnet, die insbesondere in Sticktechnik in der Auflage 12 ausgestaltet sind. In der dargestellten Form bilden sie zur Wundoberfläche hin flache bis halbrunde Erhöhungen, Reizpunkte 13, und können auf der von der Wundoberfläche abgewandten Seite ebenfalls überstehen. Die Reizpunkte 13 können alle jeweils die gleiche Grösse aufweisen oder jeweils einzeln oder in Gruppen unterschiedlich gross sein. Der Begriff Grösse bezieht sich hier sowohl auf die Höhe über und unter der Fläche des Gesticks als auch auf die Fläche in der Draufsicht der Figuren. Es können Gradientenverläufe in der Grösse vorgesehen sein, beispielsweise mit den flächenmässig grössten und in der Dicke kleinsten Reizpunkten 13 in der Mitte eines Gesticks und den flächenmässig kleinsten und in der Dicke grössten Reizpunkten 13 an den Rändern des Gesticks. Es ist auch jede andere Kombination von Dicken und Flächenverläufen einsetzbar.

Damit ist in Wundnähe eine Stickstruktur 12 und damit eine angiopolare Schicht vorgesehen. Eine angiopolare Schicht ist eine Schicht, die die gezielte orientierte Einsprossung von Blutgefässen in eine Struktur und damit eine Beeinflussung der Dichte und Ausrichtung der Blutgefässe im regenerierenden Gewebe gestattet. Mit dieser Stickstruktur 12 werden morphologische Merkmale in die Wundbehandlung eingeführt, die eine gerichtete Angiogenese innerhalb des Gerüstes induzieren und stimulieren und damit die physiologische Basis für die Gewebserneuerung bilden.

Die textile Architektur 11 und 12 schafft den optimalen mechanischen Schutz, bildet ein Reservoir für Exsudate und gestattet eine optimale Feuchtigkeits- und Gastransportsteuerung.

Mit der Stickereitechnik werden hocharchitekturierte dreidimensionale Textilstrukturen erhalten, die benötigt werden, um strukturelle Funktionen, z.B. Porenmuster, zum Aufbau einer Angiogenese einzubinden. Die Stickereitechnik gestattet dabei einen beliebigen Einsatz von Materialien in Basisgeweben.

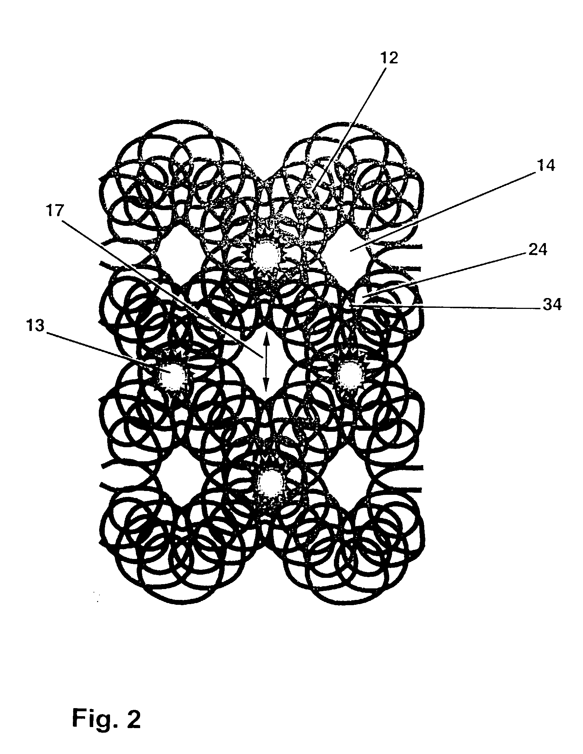

Die Fig. 2 zeigt in schematischer Weise eine Draufsicht auf einen Ausschnitt der gestickten Oberfläche 12 eines Medizinalartikels 1 gemäss der Erfindung. Die mit dem Bezugszeichen 14 bezeichneten Strukturen sind im Stickmuster vorgesehene Öffnungen, die hier im wesentlichen rautenförmig sind. Bei anderen Ausgestaltungen können diese Formen auch rechteckig, rund, elliptisch sein oder eine andere Form aufweisen.

Zur positiven Anregung der Angiogenese ist es insbesondere vorteilhaft, dass die in bezug auf die Kompresse 1 mittigen Öffnungen die grösste Durchbruchsfläche haben und entsprechende Kavitäten ausbilden. Insofern wird in dem Ausführungsbeispiel entsprechend der Fig. 2 ein Gradient vorgesehen, mit welchem sich die Durchmesser 17 der vorgesehenen Öffnungen von der Mitte her zu den Rändern hin verkleinern.

Die Öffnungen 14 sind im dargestellten Ausführungsbeispiel regelmässig angeordnet. Die Sticktechnologie gestattet ebenfalls die unregelmässige Anordnung der Öffnungen 14 entsprechend von weiteren Vorgaben, insbesondere mit einer Variation der Grösse.

Das in Verbindung mit der Fig. 1 erwähnte, hinter der gestickten Oberfläche 12 liegende Gewebe 11 wirkt als Abstandhalter und verteilt bei einer Belastung das Gewicht, um einem Dekubitus vorzubeugen. Die vorbestimmten Lochquerschnitte 17 weisen eine Grösse auf, die eine für einen Blutgerinnungspfropfen geeignete Kavität ausbilden. Sie sind damit eine Unterstützung für das geweberegenerierende Element.

Die gestickte Oberfläche, von der in der Fig. 3 ein Ausschnitt des Bereichs einer Pore 14 nach Fig. 2 zu sehen ist, verfügt neben den Durchbrüchen oder Poren 14 über mesoskopische Öffnungen 24.

Die makroskopischen Durchbrüche oder Poren 14 entstehen durch mehrere Bindungen und weisen eine Grössenordnung von 1 bis 2 Millimeter Kantenlänge auf. Sie dienen für das Einwachsen von Gewebepfropfen und als Reservoir für das Blutkoagulum aus der angefrischten blutenden Wunde.

Die mesoskopischen Öffnungen 24 gestatten das Einwachsen von einzelnen Blutgefässstämmen und haben eine Grösse von ungefähr 100 bis 500 Mikrometer. Diese entstehen durch Abbindungen von zwei Garnelementen.

Weiterhin sind hier in schematischer Darstellung mikroskopische Öffnungen 34 mit einem Durchmesser im Bereich von 5 bis 50 Mikrometer vorhanden, welche das Einwachsen von Zellen und Zellverbänden allenfalls noch mit Kapillarien ermöglichen. Diese Öffnungen 34 bestehen zwischen verschiedenen Filamenten.

In einem nochmals kleineren Massstab sind zwischen einzelnen Filamenten kleine Kavitäten im Bereich von 0,5 bis 5 Mikrometer festzustellen, in denen nur noch extrazelluläre Matrix, beispielsweise Kollagenmaterial, abgelagert werden kann.

Die Öffnungen 14, 24 und 34 bilden Gruppen von Lochmustern. Dabei sind die Öffnungen 14, die Öffnungen 24 und die Öffnungen 34 jeweils in Beziehung zu einer weiteren Gruppe von Öffnungen um einen Faktor von ungefähr mindestens 5 grösser bzw. kleiner. Innerhalb jeder Gruppe können die Öffnungen in gewissem Masse gleich gross oder unterschiedlich gross sein. Die Verteilung kann regelmässig oder auch zufällig in dem Sinne sein, dass eine Vorrichtung zum Sticken eines textilen Materials die zufällige Verteilung der Öffnungen auf der Gesamtfläche des textilen Materials mit Hilfe eines Zufallszahlengenerators steuert.

Mit dem Bezugszeichen 13 ist ein Stickpunkt dargestellt, der im dargestellten Ausführungsbeispiel jeweils zwischen zwei Kanten der Rautenöffnungen 14 liegt. Dieser Stickpunkt 13 ist in bezug auf die Zeichenebene und damit die Ebene der gestickten Auflage 12 dreidimensional und verfügt insbesondere über einen bis zu 3 bis 5 mm überstehenden Anteil. Dieser ist im dargestellten Ausführungsbeispiel fast halbkugelförmig ausgestaltet, kann jedoch auch andere dreidimensionale Strukturen annehmen.

Beispielhaft kann dieser Stickpunkt auch in der zum Abstandsgewirk hinweisenden Seite dreidimensional ausgestaltet sein, um insbesondere ein Widerlager zu bilden.

Die Regelmässigkeit des in den Figuren dargestellten Gesticks ist im Gegensatz zu bekannten Gewirken nicht systembedingt, sondern kann entsprechend dem Einsatz aufgrund der Sticktechnologie beliebig verändert werden. So sind Abfolgen von grossen und kleinen Durchbrüchen 14 möglich. Diese können, wie in der Fig. 2 dargestellt, einen Gradienten aufweisen. Die Abfolge von Stickpunkten 13 und Öffnungen 14 ist rein funktionell vorgegeben und nicht durch die Herstellungstechnologie des textilen Gewebes bedingt.

Ferner ist es möglich, dass die Durchbrüche oder Poren 14 von einem durchgehenden Faden 18 entsprechend der Fig. 3 überspannt werden, der beispielsweise bei dem Sticken der Knoten 13 von Knoten zu Knoten läuft.

Das Zusammenspiel der verschiedenen Lochgrössen der Öffnungen 14, 24, 34, ... beeinflusst in günstiger Weise das Einsprossen von Blutgefässen, die sogenannte Angiogenese. Hauptsprossen haben dabei eine Grösse von 0,5 bis 1 mm. Durch die Stickpunkte ist es möglich, in dem Wundgrund eine mechanische Reizung durchzuführen, was eine vorteilhafte Ausgestaltung des gestickten Kompressenmaterials gibt.

Als Garne können Monofilamente, Multifilamente oder Mischungen aus diesen beim Stickprozess verwendet werden. Durch die Garnwahl sowie die Vorgabe eines bestimmten Musters kann die Steifigkeit des Gestickes festgelegt werden. Dabei ist gegenüber einem Gewirk von Vorteil, dass sich der Faden nicht in der Bindung verschieben kann, das heisst die mechanischen Eigenschaften des Gesticks sind durch die Anordnungen der Bindungen definiert und werden durch die Einlagerung von Exsudat oder extrazellulärer Matrix in den Faden, das zu einem Verkleben der Bindungen führt, kaum beeinflusst. Bei Gewirken hingegen sind die mechanischen Eigenschaften im wesentlichen durch die Verschiebbarkeit des Fadens durch die offene Bindung gegeben. Damit führt ein verklebendes Exsudat zu einer Steigerung der Steifigkeit des Textils um unter Umständen weit mehr als eine Grössenordnung. Dies ist ein erheblicher Nachteil für das Medizinprodukt, da damit die mechanischen Eigenschaften, welche für seine medizinische Funktionalität entscheidend sind, nicht mehr kontrolliert werden können. Versteifungen können lokale Belastungszustände erzeugen, welche bis zur lokalen Gewebenekrose führen können.

Neben dem Einsatz des gestickten Elementes auf einer textilen Grundlage wie einer Kompresse, können auch andere Einsatzmöglichkeiten vorgesehen sein. Hierbei kann es sich um den Einsatz des gestickten Oberflächenmaterials auf keramischem oder metallischem Grund oder anderen wundbehandelnden Elementen vorgesehen sein. Durch die Sticktechnik ist es möglich, für den Einzelfall geeignete Oberflächenelemente herzustellen.

## Patentansprüche

1. Medizinalprodukt (1) mit einem textilen Bestandteil, der eine Oberfläche (12) bildet, **dadurch gekennzeichnet,** dass die Oberfläche (12) eine Vielzahl von Öffnungen (14, 24, 34) aufweist, wobei mindestens zwei Lochmuster mit Gruppen von Öffnungen (14, 24, 34) bestehen, wobei der Durchmesser einer Öffnung (14; 24) von einem Lochmuster zu dem Durchmesser einer Öffnung (24; 14 bzw. 34) eines anderen Lochmusters jeweils um ungefähr mindestens einen Faktor 5 voneinander abweicht.

2. Produkt nach Anspruch 1, dadurch gekennzeichnet, dass die textile Oberfläche (12) aus einem Gestick hergestellt ist.

3. Produkt nach Anspruch 2, dadurch gekennzeichnet, dass das Gestick Monofilamente und/oder Multifilamente und/oder Stapelfasergarne aus natürlichen, synthetischen, anorganischen und mineralischen Rohstoffen umfasst.

4. Produkt nach einem der vorstehenden Ansprüche, dadurch gekennzeichnet, dass das im Durchmesser seiner Öffnungen (14) grösste Lochmuster und das im Durchmesser seiner Öffnungen (24) zweitgrösste Lochmuster regelmässig sind.

5. Produkt nach einem der vorstehenden Ansprüche, dadurch gekennzeichnet, dass das Lochmuster mit den grössten Durchmessern in der Mitte des Produktes die grösste Öffnungsfläche (17) aufweist und gegenüber den Rändern des Produktes in einem Gradienten abnimmt.

6. Produkt nach einem der vorstehenden Ansprüche, dadurch gekennzeichnet, dass eine Vielzahl von Reizpunkten (13) zwischen den Öffnungen (14) vorgesehen ist.

7. Produkt nach Anspruch 6, dadurch gekennzeichnet, dass die Oberfläche des Reizpunktes (13) der Oberfläche des Lochmusters mit den grössten Durchmessern (14) entspricht.

8. Produkt nach Anspruch 6 oder 7, dadurch gekennzeichnet, dass jeder Reizpunkt (13) eine Höhe bis zu 5 Millimeter aufweist und/oder halbkugelförmig ausgestaltet ist.

9. Produkt nach einem der vorstehenden Ansprüche, dadurch gekennzeichnet, dass der Reizpunkt (13) auch in der wundabgewandten Seite über die Stickebene des textilen Produktes (12) hinaussteht.

10. Produkt nach einem der vorstehenden Ansprüche, dadurch gekennzeichnet, dass es eine Kompresse, ein Herniennetz, ein Bauchdeckenersatz, ein künstliches Band oder ein Augmentationstextil für Anwendungen im Dentalbereich ist.
